# EUROPEAN PATENT SPECIFICATION

(11) **EP 3 287 080 B1**
(45) Date of publication and mention of the grant of the patent: **31.08.2022**
(21) Application number: 16783084.3
(22) Date of filing: 14.04.2016
(51) Int. Cl.: A61B 8/13, G01N 29/34, A61B 5/00

(54) **PHOTOACOUSTIC WAVE DETECTING DEVICE, AND PHOTOACOUSTIC IMAGING DEVICE**
FOTOAKUSTISCHER WELLENDETEKTOR UND FOTOAKUSTISCHE BILDGEBUNGSVORRICHTUNG
DISPOSITIF DÉTECTEUR D'ONDE PHOTO-ACOUSTIQUE, ET DISPOSITIF IMAGEUR PHOTO-ACOUSTIQUE

(30) Priority: 23.04.2015 JP 2015088603
(43) Date of publication of application: 28.02.2018
(73) Proprietor: Yokogawa Electric Corporation, Musashino-shi Tokyo 180-8750 (JP)
(72) Inventor: SANGU Hiroyuki, Musashino-shi Tokyo 180-8750 (JP)
(74) Representative: Henkel & Partner mbB
(86) International application number: PCT/JP2016/061992
(87) International publication number: WO 2016/171063

(56) References cited:
- WO-A1-2014/118781
- JP-A- 2005 224 399
- JP-A- 2013 106 874
- JP-A- 2013 518 673
- US-A- 5 781 294
- US-A1- 2006 262 316

## Description

### TECHNICAL FIELD

The present invention relates to a photoacoustic wave detecting device configured to illuminate light to an observation target such as a body tissue and to detect an acoustic wave generated from the observation target by the illuminated light, and a photoacoustic imaging device using the photoacoustic wave detecting device.

### RELATED ART

In recent years, a photoacoustic imaging technology using a photoacoustic effect has been attracted attention as a method of imaging (visualizing) an inside of a living body. The photoacoustic effect is a phenomenon that molecules having absorbed the light energy releases heat and an acoustic wave is generated as a result of volume expansion due to the heat. Since the acoustic wave can be long-range propagated in vivo, the photoacoustic imaging can visualize a deep portion of the living body with high contrast.

For example, non-Patent Document 1 discloses illuminating a short pulse laser to a body tissue and detecting an acoustic wave, which is generated as thermal expansion is instantaneously caused in the body tissue. Since it is possible to know from what a depth the acoustic wave is generated by arrival time of the acoustic wave, it is possible to perform internal imaging on the basis of the detected acoustic wave.

Also, non-Patent Document 2 discloses performing internal imaging by illuminating a normal inexpensive continuous wave laser to a body tissue, instead of the short pulse laser. In this disclosure, photic stimulus is continuously applied to the body tissue by the continuous wave laser, to which intensity modulation of an ultrasonic band of MHz has been applied, instead of causing the instantaneous thermal expansion in the body tissue by the short pulse laser, so that the acoustic wave of the same band generated resultantly is detected to perform the imaging. According to the continuous wave laser, it is not possible to determine a depth by the arrival time of the acoustic wave. Therefore, it is necessary to provide a large arch-shaped acoustic wave measuring device for separating the acoustic waves generated from the various depths of the body tissue.

In the technologies disclosed in non-Patent Document 1 and non-Patent Document 2, a resolution in a depth direction (an optical axis direction) is determined by a property of the acoustic wave. For this reason, it is difficult to implement the high resolution.

In contrast, Patent Document 1 discloses a technology of improving the resolution in the depth direction by using a multiphoton excitation phenomenon. According to this technology, the resolution in the depth direction is improved using a property of the light referred to as multiphoton excitation, not the property of the acoustic wave.

### Citation List

### Patent Documents

Patent Document 1: JP-A-2011-45514

### Non-Patent Documents

Non-Patent Document 1: "Second-generation optical-resolution photoacoustic microscopy with improved sensitivity and speed", Song Hu, Konstantin Maslov, Lihong V. Wang, 1134-1136, OPTICS LETTERS/Vol. 36, No. 7/ April 1, 2011

Non-Patent Document 2: "Photoacoustic imaging of biological tissue with intensity- modulated continuous-wave laser", Konstantin Maslov, Lihong V. Wang, Journal of Biomedical Optics 13(2), 024006 (March/April 2008)

Further prior art consists in US 5 781 294 A and US 2006/262316 A1.

US 5 781 294 A discloses a method and an apparatus for detecting a photoacoustic signal which irradiate an excitation light beam, modulated by a desired frequency, simultaneously to a plurality of points being measured on a surface of a sample, irradiate the excitation light and a probe light simultaneously to the plurality of the points being measured, detect an interference light of a reflected light beam of the probe light and a specified reference light with a detector made up of a plurality of photoelectric converting elements corresponding to the respective points being measured, the detector being in conjugate relation with the surface of the sample, detect a thermal distortion of the frequency component equal to the intensity-modulated frequency at the plurality of the points being measured from the interference light intensity signal detected by the detector, and detect information relative to the surface and the subsurface of the measuring points on the sample from the thermal distortion of the frequency component. A plurality of measuring points on a specimen are excited simultaneously with an intensity-modulated flat light beam and the thermal expansion displacements of the measuring points are detected simultaneously by interference between a flat probe light beam and a flat reference light beam to detect photothermal displacement signals representing the photothermal displacements of the plurality of measuring points simultaneously.

US 2006/262316 A1 discloses a system of using an interferometer, in combination with a laser, and a detector to determine absorptive characteristics of a material under test. The operation of the interferometer allows for determination of the wavelength of the laser beam and for determining relative changes in the wavelength of the laser beam. A method for using a laser source and an interferometer to determine characteristics of a material under test is also provided.

### SUMMARY OF THE INVENTION

### Problems To Be Solved

According to the technology disclosed in Patent Document 1, it is possible to improve the resolution in the depth direction. However, it is necessary to provide a high-priced pulse laser so as to generate the multiphoton excitation.

For this reason, there is a need for a technology for implementing a high-resolution photoacoustic imaging device at low cost. It is therefore an object of the present invention to implement a high-resolution photoacoustic imaging device at low cost.

### Means for Solving Problems

In order to achieve the above object, there is provided a photoacoustic imaging device as set out in independent claim 1. Advantageous developments are defined in the dependent claims.

### Effects of the Invention

According to the present invention, it is possible to provide the high-resolution photoacoustic imaging device at low cost.

### BRIEF DESCRIPTION OF THE DRAWINGS

FIG. 1 is a block diagram depicting a configuration of a photoacoustic imaging device in accordance with an illustrative embodiment.
FIG. 2 is a block diagram depicting a configuration of a control device.
FIG. 3 pictorially depicts an aspect around a focus of a variable focus lens.

### DETAILED DESCRIPTION OF ILLUSTRATIVE EMBODIMENTS

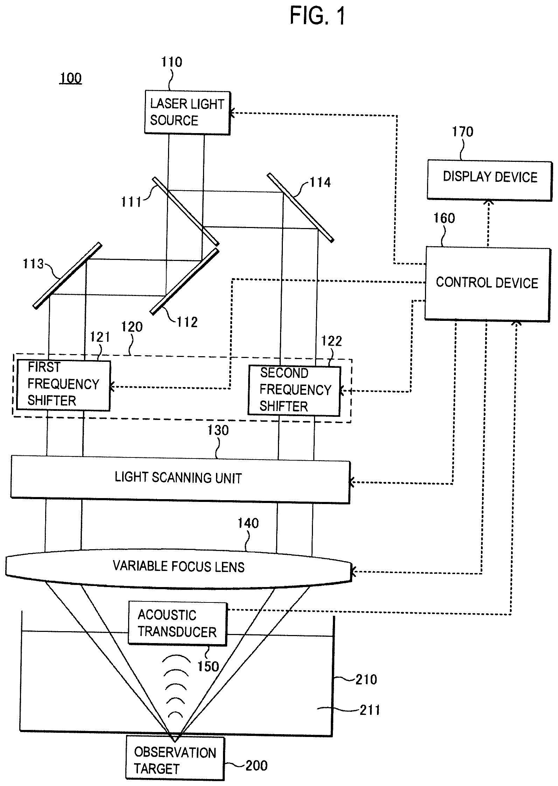

An illustrative embodiment of the present invention will be described with reference to the drawings. FIG. 1 is a block diagram depicting a configuration of a photoacoustic imaging device 100 to which a photoacoustic wave detecting device of an illustrative embodiment is applied.

As shown in FIG. 1, the photoacoustic imaging device 100 is a device configured to perform internal imaging of an observation target 200 such as a body tissue, and includes a laser light source 110, a beam splitter 111, a mirror 112, a mirror 113, a mirror 114, a first frequency shifter 121, a second frequency shifter 122, a light scanning unit 130, a variable focus lens 140, an acoustic transducer 150, a control device 160, and a display device 170.

The laser light source 110 is a light source configured to emit parallel laser lights. The laser light source 110 may be configured by a semiconductor laser light source configured to emit continuous lights, for example. A wavelength of the light emitted by the laser light source 110 is selected as a wavelength that is to be highly absorbed in the observation target 200. For example, when performing imaging of a blood vessel, which is the observation target 200, it is preferably to select a wavelength near 532nm which hemoglobin highly absorbs.

The beam splitter 111 configured to bisect the light is disposed in a light emission direction of the laser light source 110. One split light by the beam splitter 111 is guided to the first frequency shifter 121 by the mirror 112 and the mirror 113, and the other split light is guided to the second frequency shifter 122 by the mirror 114. In the meantime, the first frequency shifter 121 and the second frequency shifter 122 configure a frequency shifting unit 120, and function as an optical physical property shifting unit configured to change a physical property of the light.

In the shown example, the light is split into light in a straight direction and light in a 90° direction at the beam splitter 111 disposed at 45° relative to the emission direction of the laser light source 110, and the light in the straight direction is traveled in the same direction as the emission direction of the laser light source 110 and is then incident on the first frequency shifter 121 by the mirror 112 and the mirror 113 inclined at 45°. Also, the light split in the 90° direction at the beam splitter 111 is traveled in the same direction as the emission direction of the laser light source 110 by the mirror 114 inclined at 45° and is then incident on the second frequency shifter 122.

The arrangement and configurations of the beam splitter 111 and the respective mirrors 112 to 114 are not limited to the shown example but enable the respective lights split at the beam splitter 111 to be incident on the first frequency shifter 121 and the second frequency shifter 122 in the same direction.

The first frequency shifter 121 and the second frequency shifter 122 are configured by acousto-optical modulators, for example, and are configured to slightly shift a frequency of light. Herein, a frequency shift amount of the first frequency shifter 121 and a frequency shift amount of the second frequency shifter 122 are made different. One frequency shift amount may be zero.

The two split lights of which at least one frequency is shifted by the frequency shifting unit 120 travel in the same direction, are incident on the variable focus lens 140 via the light scanning unit 130, and form a focus, respectively. Herein, the two lights are configured to be incident in parallel with an optical axis of the variable focus lens 140. For this reason, the two lights intersect with each other at a focus of the variable focus lens 140.

The light scanning unit 130 includes a Galvano mirror and the like, and is configured to scan the focus in at least one direction on a plane perpendicular to the optical axis of the variable focus lens 140.

The variable focus lens 140 is a lens capable of changing a focal distance. It is preferably to select a lens capable of changing a focal distance at high speed.

In front of the variable focus lens 140, a medium receptacle 210 in which a medium 210 such as water for transmitting an acoustic wave is accommodated is disposed. An observation target 200 such as a body tissue is fixed to a bottom surface of the medium receptacle 210. As shown in FIG. 1, a variable focus of the variable focus lens 140 is located in the observation target 200.

Also, the acoustic transducer 150 functioning as an acoustic detecting unit is disposed in the medium 211 in the medium receptacle 210. The acoustic transducer 150 is configured to detect an acoustic wave, which is generated from the observation target 200, and to convert the same into an electric signal.

The beam splitter 111, the mirrors 112 to 114, the frequency shifting unit 120, the light scanning unit 130, the variable focus lens 140, and the acoustic transducer 150 configure the photoacoustic wave detecting device of the illustrative embodiment.

The control device 160 is a block configured to control the laser light source 110, the frequency shifting unit 120, the light scanning unit 130, the variable focus lens 140, and the acoustic transducer 150, and is connected to the display device 170.

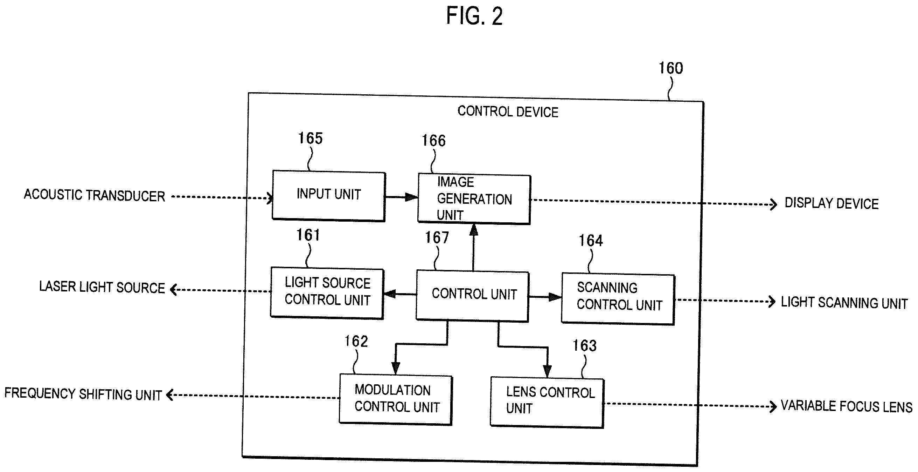

FIG. 2 is a block diagram depicting a configuration of the control device 160. As shown in FIG. 2, the control device 160 includes a light source control unit 161, a modulation control unit 162, a lens control unit 163, a scanning control unit 164, an input unit 165, an image generation unit 166, and a control unit 167.

The light source control unit 161 is configured to control light emission processing of the laser light source 110. The modulation control unit 162 is configured to control a frequency shift amount of the frequency shifting unit 120. The lens control unit 163 is configured to control a focal distance of the variable focus lens 140. The scanning control unit 164 is configured to control an operating position of the light scanning unit 130. The input unit 165 is configured to receive a detection signal from the acoustic transducer 150.

The image generation unit 166 is configured to generate and preserve an internal image of the observation target 200, based on the detection signal, information such as the focal position of the variable focus lens 140, and the like, and to output the same to the display device 170 and the like, as necessary.

The control unit 167 is configured to control various operations of the light source control unit 161, the modulation control unit 162, the lens control unit 163, the scanning control unit 164, and the image generation unit 166.

Subsequently, operations of the photoacoustic imaging device 100 configured as described above are described. The laser light source 110 starts to oscillate by a signal from the light source control unit 161 of the control device 160, thereby emitting the laser light. The laser light is bisected by the beam splitter 111, so that it is split into lights traveling in the straight direction and the 90° direction (the rightward direction in FIG. 1).

The light traveling in the straight direction is guided to the first frequency shifter 121 by the mirror 112 and the mirror 113, and the light traveling in the 90° direction is guided to the second frequency shifter 122 by the mirror 114.

Herein, the first frequency shifter 121 is set to shift a frequency of the light by 10MHz and the second frequency shifter 122 is set to shift a frequency of the light by 12MHz by the modulation control unit 162 of the control device 160.

The lights shifted to the different frequencies by the first frequency shifter 121 and the second frequency shifter 122 pass through the light scanning unit 130 and are then incident on the variable focus lens 140. Then, the respective lights form a focus by the operation of the variable focus lens 140.

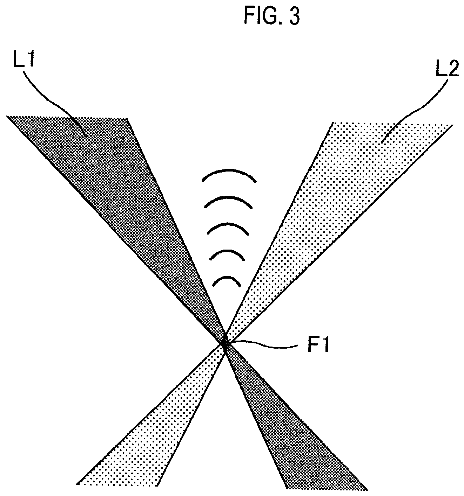

FIG. 3 pictorially depicts an aspect around the focus of the variable focus lens 140. In FIG. 3, the light having passed through the first frequency shifter 121 is denoted with LI, and the light having passed through the second frequency shifter 122 is denoted with L2.

Since the two lights LI, L2 are incident in parallel with the optical axis of the variable focus lens 140, the lights intersect at the focus of the variable focus lens 140 to form an intersection region F1. Upon the observation, the intersection region F1 is located in the observation target 200.

Since the light L1 and the light L2 have slightly different frequencies, the light L1 and the light L2 interfere with each other in the intersection region F1, so that intensity modulation of the difference frequency is made. In this example, since the difference frequency is 2MHz, the intensity modulation of 2MHz is made. That is, the 2 MHz intensity-modulated light is illuminated only in the intersection region F1.

Thereby, when the observation target 200 contains abundantly a material such as hemoglobin in which the light of a wavelength emitted from the laser light source 110 is highly absorbed, photic stimulus of 2MHz is absorbed and converted into thermal stimulus, so that the observation target 200 can be periodically warmed and thus causes thermal expansion. By the periodic thermal expansion, an acoustic wave of 2MHz is generated from a region corresponding to the intersection region F1. Herein, the more the absorption of light, the conversion amount into the thermal stimulus is increased, so that the acoustic wave to be generated becomes stronger.

The acoustic wave generated in the observation target 200 is propagated in the medium 211 accommodated in the medium receptacle 210, and is detected and converted into a detection signal of an electric signal by the acoustic transducer 150. At this time, a band near 2MHz, which is the difference frequency, is extracted by a filter or the like (not shown). The detection signal is input and temporarily preserved in the input unit 165 of the control device 160.

The lens control unit 163 of the control device 160 changes the focal distance of the variable focus lens 140, so that the intersection region F1, which is an acoustic generation region, can be moved in a depth direction. For this reason, the control device 160 can acquire the detection signal of the acoustic wave while changing a position of the intersection region F1 in the depth direction. In general, since the variable focus lens 140 can change the focal distance at high speed, it is possible to perform the scanning in the depth direction at high speed.

Also, the scanning control unit 164 of the control device 160 controls the light scanning unit 130, so that the intersection region F1 can be moved on a plane perpendicular to the optical axis of the variable focus lens 140. Herein, the intersection region is moved in one direction (horizontal direction) but may be moved in two directions (horizontal and vertical directions).

For this reason, the control device 160 can acquire a distribution of magnitudes of the acoustic waves on a cross-section of the observation target 200. This distribution indicates a distribution of magnitudes of the absorption of light of a wavelength emitted from the laser light source 110.

The image generation unit 166 generates, preserves and displays a cross-sectional image of the observation target 200 on the display device 170, based on the detection signal temporarily preserved by the input unit 165, and the focal distance information of the variable focus lens 140 and the scanning position information of the light scanning unit 130 acquired from the control unit 167.

The operations of the photoacoustic imaging device 100 of the illustrative embodiment have been described. In the photoacoustic imaging device 100, the acoustic wave is generated only in the limited region of the intersection region F1 in which the two lights intersect at the focus. For this reason, it is possible to obtain the high resolution. Also, since the laser light source 110 has only to emit the continuous waves and a special pulse laser is not required, it is possible to implement the photoacoustic imaging device at low cost.

Also, since the scanning in the depth direction is performed by changing the focal distance of the variable focus lens 140, it is possible to easily specify a position in the depth direction at which the acoustic wave is generated.

Meanwhile, in the above illustrative embodiment, the frequency shifting unit 120 including the first frequency shifter 121 and the second frequency shifter 122 is used. However, since it is sufficient to shift the frequency of at least one split light, one of the first frequency shifter 121 and the second frequency shifter 122 may be omitted.

In the above illustrative embodiment, the intensity-modulated lights of the difference frequency of the split lights are illuminated only in the intersection region F1. However, the image may be generated by changing the difference frequency. Thereby, it is possible to obtain an acoustic wave image corresponding to the difference frequency. For example, it is possible to obtain information such as an elastic property of the observation target 200, for example an acoustic wave enlarged at a specific frequency.

Also, in the above illustrative embodiment, the frequencies of the split lights are shifted to cause the interference in the intersection region F1. However, the other physical property of the split light may be shifted inasmuch as it is possible to generate an intensity-modulated light of a specific frequency by interference. For example, phase modulation of a constant frequency may be applied to the two split lights or one of the split lights by using an electro-optical modulator, instead of the acousto-optical element, so that an intensity-modulated light of a specific frequency by interference may be generated in the intersection region F1.

The above simply describes the specific favorable illustrative embodiment for illustrating and exemplifying the present invention. Therefore, the present invention is not limited to the illustrative embodiment and includes more changes and modifications that can be made without departing from the gist of the present invention.

### Description of Reference Numerals

100...photoacoustic imaging device, 110...laser light source 111...beam splitter, 112...mirror, 113...mirror, 114...mirror 120...frequency shifting unit, 121 ...first frequency shifter 122...second frequency shifter, 130...light scanning unit 140...variable focus lens, 150...acoustic transducer, 160...control device 161...light source control unit, 162...modulation control unit 163...lens control unit, 164...scanning control unit, 165...input unit 166...image generation unit, 167...control unit, 170...display device 200...observation target, 210...medium receptacle, 211...medium

## Claims

1. A photoacoustic imaging device (100) comprising:
a light source (110) configured to emit light;
a beam splitter (111) configured to split the light emitted from the light source (110);
an optical physical property shifting unit (120) configured to change a physical property of at least one of the split lights so that the split lights interfere with each other;
a lens (140) into which each of the split lights enters in parallel with an optical axis; and
an acoustic detecting unit (150) configured to detect an acoustic wave generated in a focal region of the lens (140) in an observation target.

2. The photoacoustic imaging device (100) according to claim 1, wherein the optical physical property shifting unit (120) is configured to shift a frequency of at least one of the split lights.

3. The photoacoustic imaging device (100) according to claim 2, wherein the acoustic detecting unit (150) is configured to detect the acoustic wave for a difference frequency band of the split lights of which at least one frequency has been shifted.

4. The photoacoustic imaging device (100) according to claim 1, wherein the optical physical property shifting unit (120) is configured to apply phase modulation of a constant frequency to at least one of the split lights.

5. The photoacoustic imaging device (100) according to one of claims 1 to 4, wherein the lens (140) is a variable focus lens.

6. The photoacoustic imaging device (100) according to one of claims 1 to 5, further comprising:
a light scanning unit (130) configured to scan the focal region of the lens (140) on a plane perpendicular to the optical axis.

7. The photoacoustic imaging device (100) according to one of claims 1 to 6, further comprising:
a control device (160) configured to generate an internal image of the observation target, based on a detection signal detected by the acoustic detecting unit (150).

## Patentansprüche

1. Eine photoakustische Abbildungsvorrichtung (100), die Folgendes aufweist:
eine Lichtquelle (110), die konfiguriert ist, um Licht zu emittieren;
einen Strahlenteiler (111), der konfiguriert ist, um das von der Lichtquelle (110) emittierte Licht zu teilen;
eine Einheit (120) zur Verschiebung optischer physikalischer Eigenschaften, die konfiguriert ist, um eine physikalische Eigenschaft von mindestens einem der geteilten Lichter so zu ändern, dass die geteilten Lichter miteinander interferieren;
eine Linse (140), in die jedes der geteilten Lichter parallel zu einer optischen Achse eintritt; und
eine akustische Erfassungseinheit (150), die konfiguriert ist, um eine akustische Welle zu erfassen, die in einem Fokusbereich der Linse (140) in einem Beobachtungsziel erzeugt wird.

2. Die photoakustische Abbildungsvorrichtung (100) nach Anspruch 1, wobei die Einheit (120) zur Verschiebung optischer physikalischer Eigenschaften konfiguriert ist, um eine Frequenz von mindestens einem der geteilten Lichter zu verschieben.

3. Die photoakustische Abbildungsvorrichtung (100) nach Anspruch 2, wobei die akustische Erfassungseinheit (150) konfiguriert ist, um die akustische Welle für ein Differenzfrequenzband der geteilten Lichter, von denen mindestens eine Frequenz verschoben wurde, zu erfassen.

4. Die photoakustische Abbildungsvorrichtung (100) nach Anspruch 1, wobei die Einheit (120) zur Verschiebung optischer physikalischer Eigenschaften (120) konfiguriert ist, um eine Phasenmodulation mit einer konstanten Frequenz auf mindestens eines der geteilten Lichter anzuwenden.

5. Die photoakustische Abbildungsvorrichtung (100) nach einem der Ansprüche 1 bis 4, wobei die Linse (140) eine Linse mit variablem Fokus ist.

6. Die photoakustische Abbildungsvorrichtung (100) nach einem der Ansprüche 1 bis 5, ferner aufweisend:
eine Lichtabtasteinheit (130), die konfiguriert ist, um den Fokusbereich der Linse (140) in einer Ebene senkrecht zu der optischen Achse abzutasten.

7. Die photoakustische Abbildungsvorrichtung (100) nach einem der Ansprüche 1 bis 6, ferner aufweisend:
eine Steuervorrichtung (160), die konfiguriert ist, um basierend auf einem von der akustischen Erfassungseinheit (150) erfassten Erfassungssignal ein internes Bild des Beobachtungsziels zu erzeugen.

## Revendications

1. Dispositif imageur photo-acoustique (100) comprenant :
une source de lumière (110) configurée pour émettre de la lumière ;
un séparateur de faisceau (111) configuré pour séparer la lumière émise par la source de lumière (110) ;
une unité optique de changement de propriété physique (120) configurée pour changer une propriété physique d'au moins l'une des lumières séparées de sorte que les lumières séparées se perturbent ;
une lentille (140) dans laquelle chacune des lumières séparées pénètre parallèlement à un axe optique ; et
une unité de détection acoustique (150) configurée pour détecter une onde acoustique générée dans une zone focale de la lentille (140) dans une cible d'observation.

2. Dispositif imageur photo-acoustique (100) selon la revendication 1, dans lequel l'unité optique de changement de propriété physique (120) est configurée pour changer une fréquence d'au moins l'une des lumières séparées.

3. Dispositif imageur photo-acoustique (100) selon la revendication 2, dans lequel l'unité de détection acoustique (150) est configurée pour détecter l'onde acoustique pour une bande de fréquences de différence des lumières séparées dont au moins une fréquence a été changée.

4. Dispositif imageur photo-acoustique (100) selon la revendication 1, dans lequel l'unité optique de changement de propriété physique (120) est configurée pour appliquer une modulation de phase d'une fréquence constante à au moins l'une des lumières séparées.

5. Dispositif imageur photo-acoustique (100) selon l'une des revendications 1 à 4, dans lequel la lentille (140) est une lentille à focale variable.

6. Dispositif imageur photo-acoustique (100) selon l'une des revendications 1 à 5, comprenant en outre :
une unité de balayage de la lumière (130) configurée pour balayer la zone focale de la lentille (140) sur un plan perpendiculaire à l'axe optique.

7. Dispositif imageur photo-acoustique (100) selon l'une des revendications 1 à 6, comprenant en outre :
un dispositif de commande (160) configuré pour générer une image interne de la cible d'observation, sur la base d'un signal de détection détecté par l'unité de détection acoustique (150).
